# EUROPÄISCHE PATENTANMELDUNG

(11) **EP 0 936 465 A2**
(43) Veröffentlichungstag der Anmeldung: **18.08.1999**
(21) Anmeldenummer: 99102427.4
(22) Anmeldetag: 09.02.1999
(51) Int. Cl.: G01N 27/49, G01N 33/20

(54) **Prüfverfahren für das Wasserstoffverhalten von Metallproben sowie Messapparatur zur Durchführung des Verfahrens**

(30) Priorität: 10.02.1998 DE 19805194
(71) Anmelder: Thyssen Krupp Stahl AG, 40211 Düsseldorf (DE)
(72) Erfinder: Nazikkol, Cetin Dr.rer.nat., 47169 Duisburg (DE); Waldöfner, Karl-Heinz, 47167 Duisburg (DE); Mittelstädt, Horst Dr.rer.nat., 44892 Bochum (DE); Wienströer, Stefan Dipl.-Chem., 48145 Münster (DE); Wiemhöfer, Hans-Dieter Prof.Dr.rer.nat., 48161 Münster (DE); Bremes, Hans-Gerd, 59075 Hamm (DE)
(74) Vertreter: Cohausz & Florack

(57) **Zusammenfassung**

Die Erfindung betrifft ein Meßverfahren und eine Meßapparatur für das Wasserstoffverhalten einer Metallprobe. Dafür wird die Beaufschlagung der Vorderseite der Metallprobe (P) mit Wasserstoff über einen protonenleitenden Festkörperelektrolyten durch Anlegen einer Spannung eingeleitet und an der Rückseite wird über einen protonen- oder sauerstoffionenleitenden Kontakt, insbesondere in Form eines Festkörperelektrolyten (F₃), ein elektrischer Meßwert in Form von Spannung oder Strom der Metallprobe (P) aufgenommen. Ein solches Meßverfahren und eine solche Meßapparatur sind vor allem geeignet, die Fischschuppenanfälligkeit von Metall beim Emaillieren zu untersuchen.

## Beschreibung

Über das Wasserstoffverhalten einer Metallprobe ist es möglich, Eigenschaften der Metallprobe festzustellen. So ist es zum Beispiel bekannt, mittels eines bestimmten Prüfverfahrens für das Wasserstoffverhalten von Feinblechen deren Eignung für das Emaillieren festzustellen. Zu den am meisten gefürchteten Emaillierfehlern zählen sogenannte Fischschuppen, weil sie nicht sofort erkennbar sind, sondern erst längere Zeit nach der Emaillierung in Erscheinung treten. Das für solche Fehler anerkannte Prüfverfahren war früher in der deutschen Norm DIN 1623-3 von 1987 und ist heute in der europäischen Norm EN 10209 von Mai 1996 festgelegt. Bei diesem Verfahren wird die Vorderseite der Stahlprobe mit einem wässrigen Elektrolyten in Kontakt gebracht. Der durch die Metallprobe diffundierende Wasserstoff wird auf der Rückseite der Metallprobe gesammelt. Mit einer vorgegebenen Formel wird ein Kennwert für die Wasserstoffdiffusion ermittelt, bei dem die Durchtrittszeit für den Wasserstoff durch die Probe unter Berücksichtigung der Dicke der Probe maßgebend ist. Dieser Kennwert ist dann ein Beurteilungskriterium für die Fischschuppenanfälligkeit der Metallprobe.

Es wurde gefunden, daß ein solches Verfahren bei herkömmlichen, unlegierten Emaillierstahlgüten eine zufriedenstellende Aussage über deren Fischschuppenanfälligkeit liefert, nicht jedoch für mit Bor oder Titan legierte Stahlqualitäten, wie IF-Stahlgüten, die sich insbesondere für Tiefziehzwecke gut eignen. Hinzu kommt, daß das bekannte Verfahren nur mit großem Aufwand durchzuführen ist, keine befriedigende Reproduzierbarkeit gewährleistet und nur bei Raumtemperatur funktioniert.

Demgegenüber wird bei einem anderen bekannten Verfahren ein protonenleitender Festkörperelektrolyt zur Messung der Wasserstoffdiffusion durch eine Metallprobe eingesetzt. Dazu ist der Festkörperelektrolyt über einen Distanzring auf der Rückseite der Metallprobe angeordnet. Wird die Konzentration des Wasserstoffs auf der Vorderseite der Metallprobe schlagartig von 1% auf 100% erhöht, dann tritt zeitverzögert in den von dem Distanzring und dem Festkörperelektrolyten gebildeten Sammelraum Wasserstoff ein. Die Wasserstoffdiffusion kann deshalb in Abhängigkeit von der Zeit gemessen werden. Dafür liegt an dem Festkörperelektrolyten eine Spannung an. Die schlagartige Änderung der Konzentration des Wasserstoffes auf der Vorderseite der Probe macht sich zeitverzögert in einem starken Stromanstieg bemerkbar. Eine Schwierigkeit bei diesem Verfahren besteht darin, den Zeitpunkt der schlagartigen Änderung der Wasserstoffkonzentration an der Vorderseite der Metallprobe zu bestimmen bzw. die Totzeiten für den Wasserstoffeintritt in die Probe möglichst klein zu halten. Diese Schwierigkeit wirkt sich nachteilig auf die Meßgenauigkeit der Durchtrittszeit aus.

Der Erfindung liegt die Aufgabe zugrunde, ein Meßverfahren und eine Meßapparatur zum Prüfen des Wasserstoffverhaltens von Metallproben zu schaffen, das universell anwendbar ist, exaktere Meßergebnisse als bekannte Verfahren liefert und einfach zu handhaben ist.

Diese Aufgabe wird erfindungsgemäß mit einem Prüfverfahren für das Wasserstoffverhalten von Metallproben gelöst, bei dem an der Rückseite der Probe über einen protonen- oder sauerstoffionenleitenden Festkörperelektrolyten der Einfluß der Beaufschlagung der Vorderseite mit Wasserstoff über einen protonenleitenden Festkörperelektrolyten durch Messung einer elektrischen Größe, und zwar der Spannung oder des Stroms, an der Rückseite nach Anlegen einer elektrischen Spannung an der Vorderseite ermittelt wird.

Die für die Durchführung dieses Prüfverfahrens vorgesehene Apparatur besteht aus einem auf der Vorderseite der Metallprobe angeordneten, einen protonenleitenden Festkörperelektrolyten umfassenden Beaufschlagungsmittel für Wasserstoff und einem elektrischen Kontaktmittel zum Anlegen einer elektrischen Spannung sowie an der Rückseite der Metallprobe aus einem protonen- oder sauerstoffionenleitenden Kontakt sowie einem daran angeschlossenen Meßinstrument für die Spannung oder den Strom.

Bei dem erfindungsgemäßen Prüfverfahren und der erfindungsgemäßen Meßapparatur ist auf einen wässrigen Elektrolyten verzichtet worden. Statt dessen wird bei Beaufschlagung der Vorderseite der Metallprobe mit Wasserstoff ein protonenleitender Festkörperelektrolyt verwendet, die Diffusion mit dem Anlegen einer elektrischen Spannung eingeleitet und an der Rückseite eine elektrische Größe gemessen. Der Aufwand ist im Vergleich zu Meßapparaturen mit wässrigem Elektrolyten an der Probenvorderseite gering. Auch liefert die Meßapparatur über die Erfassung einer elektrischen Größe genauere Meßergebnisse über die Wasserstoffdurchlässigkeit der Metallprobe, weil durch das Anlegen der Spannung an der Vorderseite sowie der Pufferung der Protonen an der Vorderseite der Metallprobe die Diffusion praktisch ohne Totzeit beim Einschalten der Spannung beginnt. Hinzu kommt, daß über die an der Vorderseite anliegende Spannung die Diffusion beeinflußt werden kann. Wie bei den bekannten Verfahren lassen sich über die ermittelten Werte für die Wasserstoffdurchlässigkeit Aussagen insbesondere über die Fischschuppenanfälligkeit der Metallprobe machen. Über die Meßwerte für die Wasserstoffdurchlässigkeit lassen sich aber auch andere Eigenschafen der Metallprobe, wie Gefügezustände, zum Beispiel in den Schweißnähten, oder die Oberflächenschichten, insbesondere Oxidschichten, der Metallprobe ermitteln. Ein besonderer Vorteil gegenüber dem bekannten Meßverfahren mit dem wässrigen Elektrolyten ist, daß die Messung auch bei wesentlich höheren Temperaturen als Raumtemperatur durchgeführt werden kann. Die Aussagefähigkeit des Meßergebnisses kann durch Kombination verschiedenartiger Meßwerte erweitert werden.

Der protonenleitende Festkörperelektrolyt kann mit einem Wasserstoff-Gas-Gemisch beaufschlagt werden, zum Beispiel in der Weise, daß vor der Vorderseite der Metallprobe eine Kammer mit einem Wasserstoff-Gas-Gemisch angeordnet ist.

Nach einer weiteren Ausgestaltung der Erfindung kann das an der Vorderseite der Probe vorgesehene Kontaktmittel eine auf dem Festkörperelektrolyten angeordnete poröse Elektrode, insbesondere aus einem Katalysator, wie Platin oder Palladium, sein.

Um den Eintritt des Wasserstoffs in die Probe und aus der Probe zu begünstigen, das heißt, die Totzeiten für die Messung der Durchtrittszeit möglichst klein zu halten, ist vorgesehen, daß unmittelbar auf der Vorderseite und/oder Rückseite der Probe eine Katalysatorschicht angeordnet ist.

Vorzugsweise ist der protonenleitende Kontakt an der Rückseite der Metallprobe als Mikrokontakt ausgebildet. Der Vorteil dieser Ausgestaltung der Erfindung liegt darin, daß je nach Größe des Mikrokontaktes die Meßzeiten erheblich verkürzt werden, weil sich dann höhere Stromdichten ergeben. Außerdem ergeben sich damit kleinere Meßflächen, die ein Scannen der Probenoberfläche ermöglichen, um zum Beispiel die Deckschicht der Rückseite örtlich untersuchen zu können.

Nach einer weiteren Ausgestaltung der Erfindung ist vorgesehen, daß der protonenleitende oder sauerstoffionenleitende Festkörperelektrolyt mit räumlichem Abstand zur Probe angeordnet und in einer mit Inertgas bespülten Kammer untergebracht ist. Diese Ausgestaltung ermöglicht die Untersuchung des Wasserstoffverhaltens der Probe unter verschiedenen Temperaturen, weil die Metallprobe wegen der räumlichen Trennung mit einer anderen Temperatur als der Festkörperelektrolyt auf der Rückseite betrieben werden kann. Eine bevorzugte Ausführung dieser Ausgestaltung besteht darin, daß die Rückseite der Metallprobe und die der mit Inertgas bespülten Kammer abgewandte Seite des Festkörperelektrolyten von einem beide Seiten nacheinander überströmenden Gasstrom miteinander verbunden sind. Dabei kann in der den Gasstrom führenden Leitung eine temperaturentkoppelnde Kapillarbrücke, insbesondere aus Quarzglas, vorgesehen sein.

Im folgenden wird die Erfindung anhand einer Zeichnung näher erläutert, die verschiedene Ausführungen einer Meßapparatur zeigt. Im einzelnen zeigen:
- Figuren 1 - 4: Meßapparaturen mit asymmetrischer oder symmetrischer Anordnung von Anschlußelektroden für eine Gleichstrom- und/oder Gleichspannungsmessung
**und**
- Figur 5: eine Meßapparatur mit räumlicher Trennung der Beaufschlagungskammer und der Empfängerkammer und Vorrichtung zum Transport des Wasserstoffs.

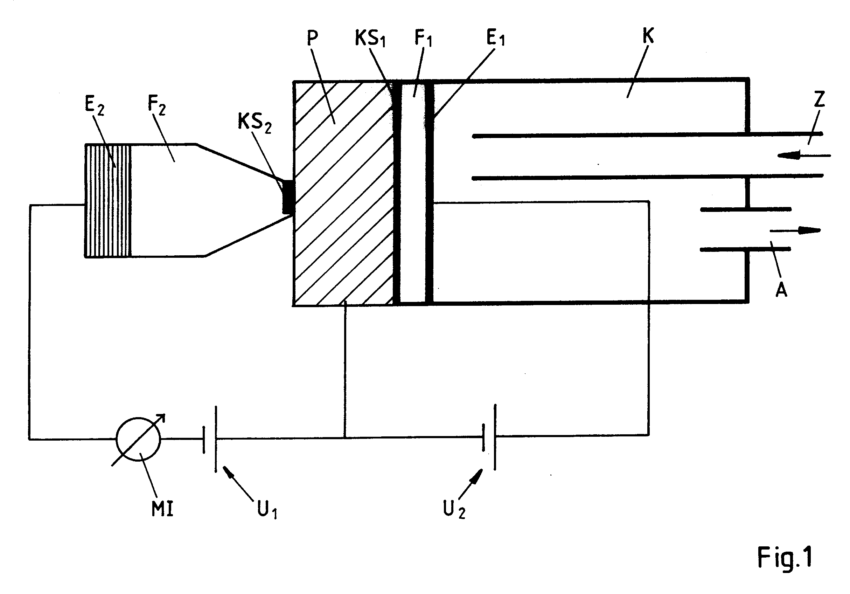

Bei den verschiedenen Meßapparaturen der Figuren 1 - 4 findet die Messung mit Gleichspannung beziehungsweise Gleichstrom statt.

Dazu ist bei der Meßapparatur der Fig. 1 auf der Vorderseite einer Metallprobe P, insbesondere über eine Zwischenschicht KS₁ aus Katalysatormaterial, z.B. Pt oder Pd, ein protonenleitender Festkörperelektrolyt F₁ vollflächig angeordnet. Auf diesem Festkörperelektrolyten F₁ ist vollflächig eine poröse Pt-Elektrode E₁ angeordnet. Dem Festkörperelektrolyten F₁ und der Elektrode E₁ ist eine mit einem Wasserstoff-Gas-Gemisch, insbesondere H2/Ar-Gemisch, gefüllte Kammer K vorgeordnet. Damit in der Kammer K genügend Wasserstoffionen für die Beaufschlagung der Vorderseite der Metallprobe P zur Verfügung stehen, findet über einen Einlaß Z und einen Auslaß A eine ständige Erneuerung des Gemisches statt.

An der Rückseite der Metallprobe P ist eine poröse PT-Elektrode E₂ mit einem protonenleitenden Mikrokontakt F₂, vorzugsweise über eine Katalysatorschicht KS₂, angeschlossen. Der Mikrokontakt F₂ ist als Festkörperelektrolyt ausgebildet.

An der Metallprobe P und der vorderseitigen Elektrode E₁ liegt eine Spannung U₂, während an der Metallprobe P und der rückseitigen Elektrode E₂ eine Spannung U₁ mit einem Strommesser MI liegt.

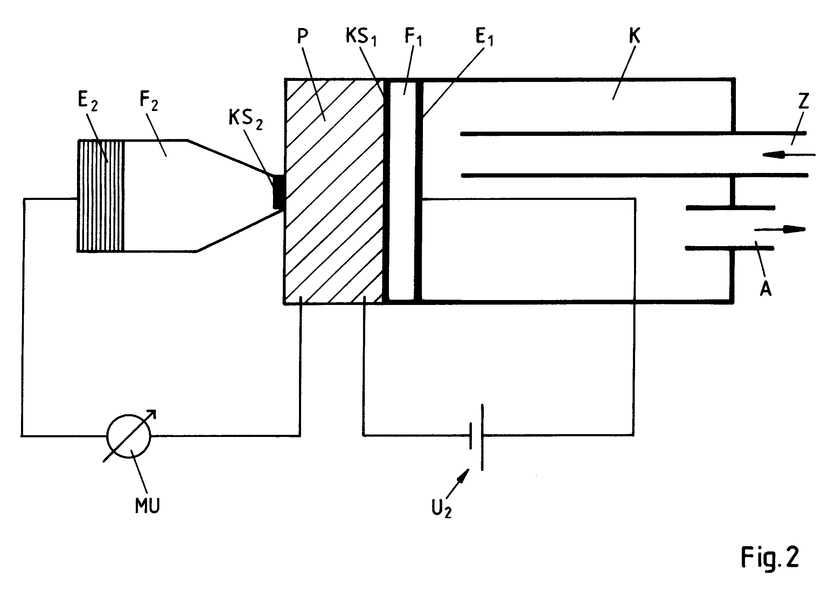

Die Meßapparatur der Fig. 2 unterscheidet sich von der der Fig. 1 nur darin, daß an der Metallprobe P und der rückseitigen Elektrode E₂ keine Spannung anliegt, sondern ein Spannungsmesser MU angeschlossen ist.

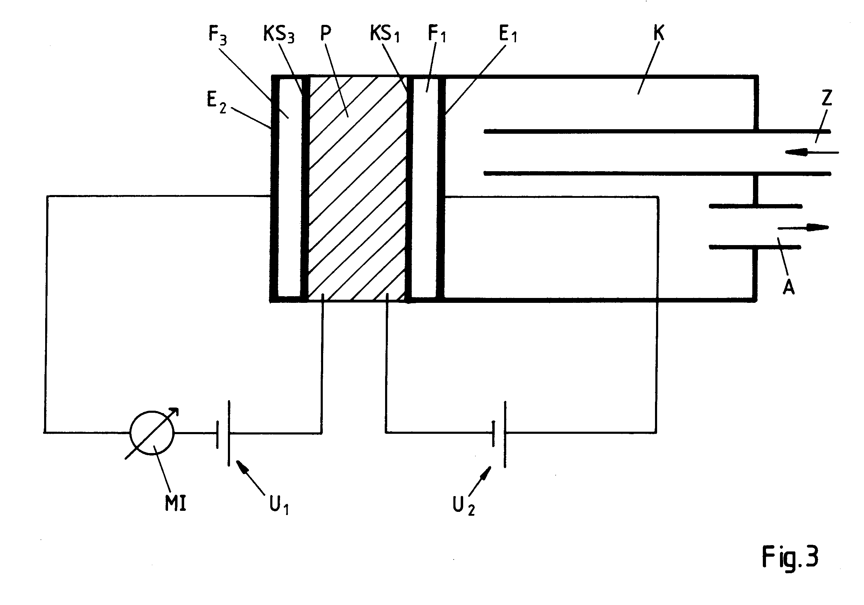

Die Meßapparatur der Fig. 3 entspricht derjenigen der Fig. 1 mit dem einzigen Unterschied, daß anstelle des protonenleitenden Mikrokontaktes F₂ ein vollflächiger protonenleitender Festkörperelektrolyt F₃ mit einer Katalysatorschicht KS₃ vorgesehen ist.

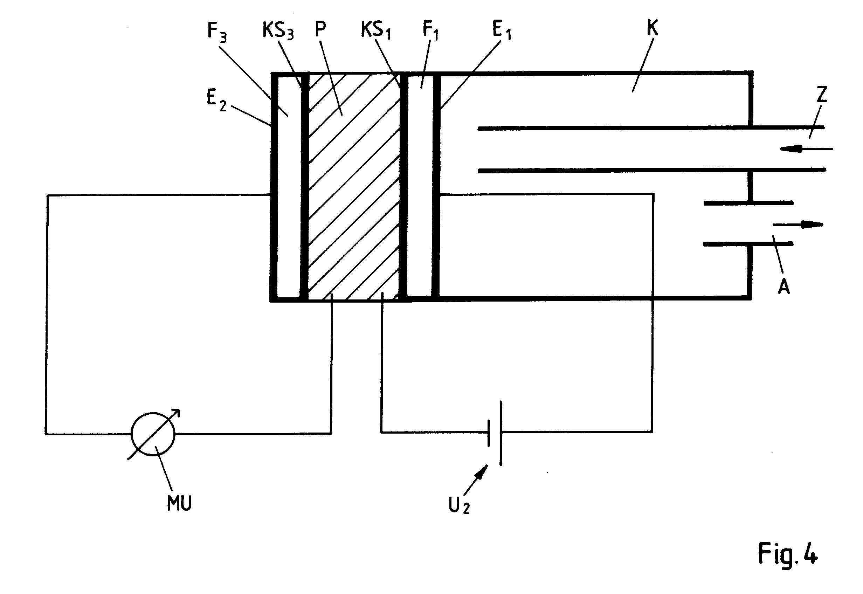

Die Meßapparatur der Fig. 4 entspricht derjenigen der Fig. 2 mit der einen Ausnahme, daß hier, wie bei der Fig. 3, ein vollflächiger protonenleitender Festkörperelektrolyt F₃ auf der Rückseite der Metallprobe P angeordnet ist.

Mit den verschiedenen Ausführungen der Meßapparaturen läßt sich über eine Gleichstrommessung und/oder stromlos über die Spannungsmessung die Zeitverzögerung des Wasserstoffverhaltens einer Metallprobe, zum Beispiel die Wasserstoffspeicherkapazität der Probe, die Anwesenheit und/oder Dicke von Oberflächenschichten, z.B. Oxidschichten, die die Wasserstoffdurchlässigkeit beeinflussen, und vieles mehr ermitteln, Eigenschaften, die für die Beurteilung der Eignung der Probe für verschiedene Verarbeitungen, wie Fischschuppenanfälligkeit, von Bedeutung sind. Eine Beschränkung bezüglich der Temperatur, unter denen die Messung stattfindet, gibt es praktisch nicht, da protonenleitende Festkörperelektrolyte Temperaturen bis in den Bereich von 1000° C erlauben. Dabei kann die Wasserstoffbeaufschlagung der Vorderseite der Metallprobe über die anliegende Spannung in Verbindung mit dem als Puffer für den Wasserstoff dienenden Festkörperelektrolyten auf der Vorderseite der Probe sehr genau gesteuert werden. Es ist deshalb möglich, die Auswirkung der Wasserstoffbeaufschlagung der Vorderseite an der Rückseite durch die zeitabhängige Strom- oder Spannungsmessung praktisch ohne das Meßergebnis verfälschende Totzeiten zu ermitteln.

Beim Einsatz von als Mikrokontakte ausgebildeten protonenleitenden Festkörperelektrolyten auf der Rückseite ergeben sich höhere Stromdichten an dieser Elektrode, die zu kürzeren Meßzeiten und größeren Empfindlichkeiten führen. Darüber hinaus bietet der Einsatz eines Mikrokontaktes die Möglichkeit, wegen der kleineren Meßflächen die Probenoberfläche zu scannen und so ortsbezogen zu untersuchen.

Für manche Anwendungen ist besonders die Ausgestaltung der Erfindung von Bedeutung, bei der der protonenleitende oder Sauerstoffionenleitende Festkörperelektrolyt mit räumlichem Abstand zur Probe angeordnet ist und in einer mit Inertgas bespülten Kammer untergebracht ist. Wegen der damit verbundenen räumlichen Trennung von Probe mit Beaufschlagungskammer auf der einen Seite und Festkörperelektrolyt mit Anschlußelektrode auf der Rückseite ist es möglich, die beiden Apparaturteile mit unterschiedlichen Temperaturen, z.B. den Apparaturteil auf der Probenrückseite mit konstanter Temperatur und den anderen Apparaturteil einschließlich der Probe bei gesteuerten unterschiedlichen Temperaturen zu betreiben, um zum Beispiel den Einfluß der Temperatur auf das Diffusionsverhalten der Probe zu untersuchen.

Bei der erfindungsgemäßen Apparatur wird der auf der Rückseite der Metallprobe P freigesetzte Wasserstoff mit dem als Transportgas wirkenden Inertgas, zum Beispiel Argon, in die Kammer K₂, das heißt zur Sensorseite, transportiert. Zur Detektion kann sowohl ein protonenleitender als auch ein oxidionenleitender Festkörperelektrolyt verwendet werden. Bei beiden Elektrolytvarianten besteht die Möglichkeit, entweder die resultierende Spannung oder den resultierenden Strom zu messen. Falls ein protonenleitender Festkörperelektrolyt verwendet wird, muß auf der gegenüberliegenden Sensorseite ein wasserstoffhaltiger Referenzgasstrom, der gegenüber dem Meßgasraum K₂ abgedichtet ist, eingestellt werden. Beim Einsatz eines oxidionenleitenden Festkörperelektrolyten ist eine Inertgasspülung an der Rückseite des Festkörperelektrolyten ausreichend. Als Sauerstoff-Referenz dient bei dieser Anordnung ein Metall-Oxidgemisch (zum Beispiel Cu/Cu₂O), das durch Kapselung einen konstanten Sauerstoffpartialdruck unabhängig vom umgebenden Gasraum erzeugt.

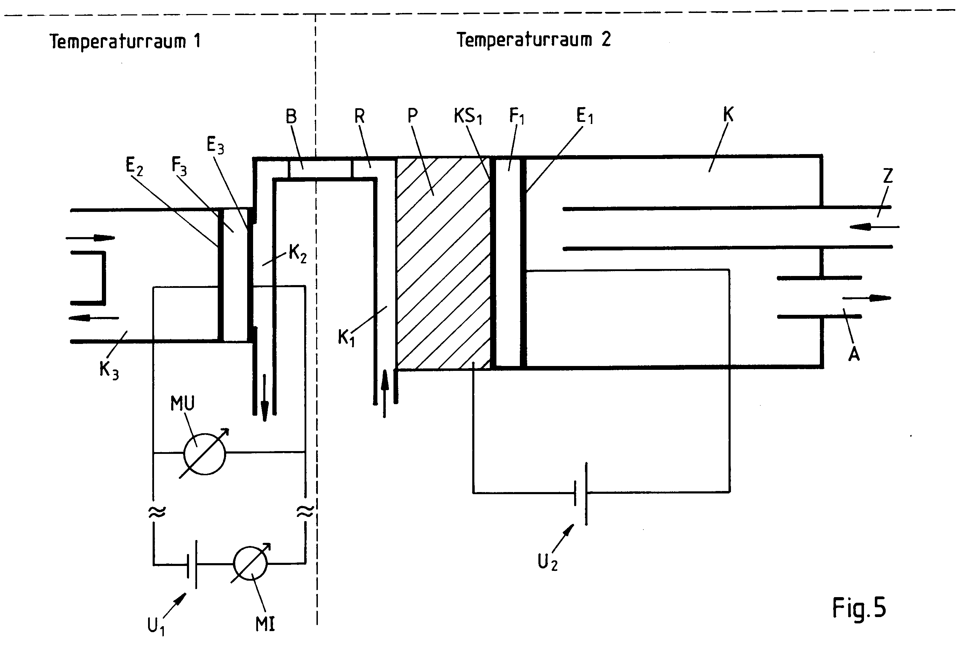

Ein Beispiel für eine solche Ausgestaltung der Erfindung ist in Figur 5 dargestellt. Bei dieser Meßapparatur sind, soweit Übereinstimmungen zur Meßapparatur der Figur 2 vorhanden sind, die gleichen Bezugszeichen verwendet. Unterschiedlich ist, daß auf der Rückseite der Metallprobe eine Kammer K₁ gasdicht angeflanscht ist. Die Kammer K₁ ist über eine Rohrleitung mit einer Kammer K₂ verbunden, die gasdicht an einer eine PT-Elektrode E₃ aufweisenden Vorderseite eines protonen- oder oxidionenleitenden Festkörperelektrolyten F₃ angeschlossen ist. Durch die Kammern K₁,K₂ wird ein Inertgas, insbesondere Ar, geleitet. Eine in der Rohrleitung R zwischen den Kammern K₁,K₂ angeordnete Kapillarbrücke B, insbesondere aus Quarzglas, dient der Temperaturtrennung zwischen der ersten Kammer K₁ und der zweiten Kammer K₂. An der ebenfalls mit einer PT-Elektrode E₂ versehenen Rückseite des Festkörperelektrolyten F₃ ist eine Kammer K₃ gasdicht angeschlossen, die von einem Ar/H₂- oder Ar-Me/MeO-Gemisch durchströmt wird. An den Elektroden E₂,E₃ liegt eine Spannung U₁ mit einem Strommesser MI oder ein Voltmeter MU an. Diese Apparatur erlaubt es, die Empfängerseite und die Metallprobe in verschiedenen Temperaturräumen 1,2 mit unterschiedlichen Temperaturen, insbesondere von Raumtemperatur bis über 600°C, zu betreiben. Damit ist der Temperatureinsatzbereich unabhängig von der Temperatur der Wasserstoffdiffusion, so daß beliebige Festkörperelektrolyten einsetzbar sind. Bei konstanter Temperatur der Empfängerseite kann die Temperatur der Beaufschlagungsseite über einen größeren Bereich, zum Beispiel auch während des Meßvorgangs, zum Beispiel nach einem vorgebenen Temperaturprogramm, variiert werden.

## Patentansprüche

1. Prüfverfahren für das Wasserstoffverhalten von Metallproben, an deren Rückseite über einen protonen- oder sauerstoffionenleitenden Festkörperelektrolyten der Einfluß der Beaufschlagung der Vorderseite mit Wasserstoff über einen protonenleitenden Festkörperelektrolyten durch Messung einer elektrischen Größe, und zwar der Spannung oder des Stroms an der Rückseite, nach dem Anlegen einer elektrischen Spannung an der Vorderseite ermittelt wird.

2. Prüfverfahren nach Anspruch 1,
**dadurch gekennzeichnet**, daß der protonenleitende Festkörperelektrolyt mit einem Wasserstoff-Gas-Gemisch beaufschlagt wird.

3. Meßapparatur für die Durchführung des Prüfverfahrens nach Anspruch 1, bestehend aus einem auf der Vorderseite der Metallprobe (P) angeordneten, einen protonenleitenden Festkörperelektrolyten (F₁) umfassenden Beaufschlagungsmittel (K) für Wasserstoff und einem elektrischen Kontaktmittel (E₁) zum Anlegen einer elektrischen Spannung und einem an der Rückseite der Metallprobe (P) vorgesehenen protonen- oder sauerstoffionenleitenden Kontakt (F₂,F₃) mit einem daran angeschlossenen Meßinstrument (MU,MI) für die Spannung oder den Strom.

4. Meßapparatur nach Anspruch 3,
**dadurch gekennzeichnet**, daß die Beaufschlagungsmittel (K) eine Kammer mit einem Wasserstoff-Gas-Gemisch (H₂/Ar) aufweisen.

5. Meßapparatur nach Anspruch 3 oder 4,
**dadurch gekennzeichnet**, daß das an der Vorderseite der Metallprobe (P) vorgesehene Kontaktmittel (E₁) eine auf dem Festkörperelektrolyten (F₁) angeordnete poröse Elektrode, insbesondere aus einem Katalysatormaterial, wie Platin oder Palladium, ist.

6. Meßapparatur nach einem der Ansprüche 3 bis 5,
**dadurch gekennzeichnet**, daß zwischen der Vorderseite der Metallprobe (P) und dem Festkörperelektrolyten (F₁) eine Katalysatorschicht (KS₁), insbesondere aus Platin oder Palladium, vorgesehen ist.

7. Meßapparatur nach einem der Ansprüche 3 bis 6,
**dadurch gekennzeichnet**, daß der an der Rückseite der Metallprobe (P) angeordnete Kontakt (F₂) als Mikrokontakt ausgebildet ist.

8. Meßapparatur nach einem der Ansprüche 3 bis 7,
**dadurch gekennzeichnet**, daß an der Rückseite der Metallprobe (P) zwischen dieser und dem protonen- oder sauerstoffionenleitenden Kontakt (E₂) eine Katalysatorschicht (KS₂) angeordnet ist.

9. Meßapparatur nach einem der Ansprüche 3 bis 8,
**dadurch gekennzeichnet**, daß der protonenleitende oder sauerstoffionenleitende Festkörperelektrolyt (F₃) mit räumlichem Abstand zur Probe (P) angeordnet und in einer mit Inertgas bespülten Kammer (K₃) untergebracht ist.

10. Meßapparatur nach Anspruch 9,
**dadurch gekennzeichnet**, daß die Rückseite der Metallprobe (P) und die der mit Inertgas bespülten Kammer (K₃) abgewandte Seite des Festkörperelektrolyten (F₃) von einem beide Seiten nacheinander überstreichenden Gasstrom miteinander verbunden sind.

11. Meßapparatur nach Anspruch 10,
**dadurch gekennzeichnet**, daß in einer den Gasstrom führenden Leitung (R) eine temperaturentkoppelnde Kapillarbrücke (B), insbesondere aus Quarzglas, vorgesehen ist.
